# EUROPEAN PATENT APPLICATION

(11) **EP 1 939 623 A1**
(43) Date of publication of application: **02.07.2008**
(21) Application number: 06811333.1
(22) Date of filing: 29.09.2006
(51) Int. Cl.: G01N 33/53, G01N 33/566, G01N 35/08, G01N 37/00

(54) **METHOD OF MEASURING BIOMOLECULAR REACTION AT ULTRAHIGH SPEED**

(30) Priority: 30.09.2005 JP 2005287990
(71) Applicant: Fuence Co., Ltd., Tokyo 150-0012 (JP)
(72) Inventor: KASE, Hiroshi, FUENCE CO. LTD., Shibuya-ku Tokyo 1500012 (JP); AOKI, Hiroyoshi, FUENCE CO. LTD., Shibuya-ku Tokyo 1500012 (JP); HARA, Tsutomu, FUENCE CO. LTD., Shibuya-ku Tokyo 1500012 (JP); NAKAMURA, Yasumasa, FUENCE CO. LTD., Shibuya-ku Tokyo 1500012 (JP); YAMAGATA, Yutaka, RIKEN, Wako-shi Saitama 3510198 (JP)
(74) Representative: Jenkins, Peter David
(86) International application number: PCT/JP2006/320006
(87) International publication number: WO 2007/037530

(57) **Abstract**

According to the present invention, a method for ultrafast and precise measurement and analysis of biomolecules using a small sample was established, and moreover, a compact and simple micro fluid device for implementing the method was provided. Using the method and the micro fluid device of the present invention, the ultrafast, instant, and precise analysis of biomolecules is possible, and there is a great deal of potential for application in the research field, manufacturing field or medical field, environmental monitoring and the like.

## Description

### TECHNICAL FIELD

The present invention relates to a method for ultrafast measurement of biomolecular reactions using a micro fluid chip system, as well as a device system to be used in the method. Using the method and device system of the present invention, it is possible to detect and measure biomolecular reactions in a small amount of time with high sensitivity.

### BACKGROUND ART

Since micro fluid chips make possible intended position measurements of biomolecular reactions with a small sample in a fast manner in a small amount of time, the micro fluid chips are becoming widely applied in areas such as medical care or drug discovery, foods, biology and biotechnology field, chemical synthesis field, environment field, and the like. Using the micro fluid chip, analysis of genome and proteins of animals, plants, or microorganisms, backup for drug discovery, clinical diagnosis, safety tests of foods, synthesis and analysis of chemical substances, environmental monitoring or the like is possible, and moreover, it makes possible an accurate, fastest possible measurement in-situ and use of the result for taking appropriate measures.

Using micro fluid chips heretofore known, it is possible to measure biomolecular reactions in a small amount of time with a small sample. The minimization of samples can be achieved by making the size of devices smaller using fine processing technology and increasing the precision in handling minute amount samples, but as for the reduction of reaction time and the increase of speed up to ultrafast levels, there are high technical barriers that still need to be overcome.

That is, biomolecular reactions such as the antigen-antibody reaction, etc. are measured using microwells or the like taking usually tens of minutes to more than one hour as the reaction time, but by using the micro fluid chip, it has become possible to detect the reaction in several minutes to several tens of minutes. However, no technology has been established that allows ultrafast and precise detection taking only an instant to several tens of seconds as the reaction time. Moreover, since for detection of an ultrafast reaction in a compact micro fluid chip, using large detection devices or large-scale control devices would reduce the benefit of using micro fluid chips for measurements and analyses by half, a simple and compact measurement system is essential. However, no ultrafast micro fluid chip system that satisfies such needs have been completed yet.

Furthermore, the inventors of the present invention have, up to this date, developed a micro fluid chip with the objective of providing a biopolymer micro chip having a structure that allows for the detection of the binding of various proteins or DNAs with other chemical substances on the micro chip and also for the recovery of the bonded substance and the identification of the same, and have reported this in Japanese Patent Application Laid-Open JP2002-243734A.

### DISCLOSURE OF INVENTION

### TECHNICAL PROBLEM

Therefore, the object of the present invention is to provide a method that makes possible the ultrafast measurement of biomolecular reactions using a micro fluid chip system and a device system to be used in the method.

In order to accomplish the above object, the present invention provides a method for measuring a biomolecular reaction at ultra-high speed comprising the steps of immobilizing biomolecule on a substrate, placing a micro fluid chip on the substrate, moving a fluid containing a molecule with an affinity for the biomolecule at a high speed on the micro fluid chip, performing a biomolecular reaction between the biomolecule and the molecule with the affinity on the micro fluid chip, and detecting the biomolecular reaction.

Moreover, the present invention provides a device system to be used for the above method characterized in that the fluid is moved at a high speed in the micro fluid chip for measuring a biomolecular reaction. The device system of the present invention preferably comprises a micro fluid chip containing a substrate with an immobilized biomolecule and a microchannel, an aspiration or pressurization circuit including an instrument for moving the fluid at a high speed, and a jig for connecting the above mentioned instrument for moving the fluid at a high speed and the above mentioned microchannel.

Using the present invention, a method for ultrafast and precise measurement and analysis of biomolecules using a small sample was established, and it became possible to provide a compact and simple micro fluid device capable of this method. There is substantial need for the ability to measure and analyze biomolecules in a very small amount of time in the intended position in the research field site, manufacturing field site or medical care field site, environmental monitoring or the like such as lab-on-a-chip and point-of-care, and it has great benefits. For example, in tailor-made medical care, or in the field sites of emergency medical care or surgery, or in the field of medical care where health status can be monitored continuously at home, ultrafast, instant, and precise analysis of biomolecules and administration of medical treatment and necessary treatment is possible, and the present invention can provide a method and device system for satisfying such needs.

### BRIEF DESCRIPTION OF THE DRAWINGS

Fig. 1 is a schematic drawing of the micro fluid chip system of the present invention.

Fig. 2 is a graph showing the relationship between the aspiration pressure by the vacuum pump and the flow rate.

Fig. 3 is a graph showing the relationship between the flow rate speed and the binding rate in the binding reaction of goat IgG and anti-goat IgG antibody (Fig. 3a) and the relationship between the flow rate speed and the binding amount of the same (Fig. 3b).

Fig. 4 is a graph showing the relationship between the flow rate speed and the binding rate in the binding reaction of avidin and biotin (Fig. 4a) and the relationship between the flow rate speed and the binding amount of the same (Fig. 4b).

Fig.5 is a figure showing the effect of high-speed rinsing on the substrate binding amount and the surface structure of the immobilized protein.

### REFERENCE SIGNS LIST

10: fluid chip outlet presser, 11: fluid chip sealing plate, 12: fluid chip guiding plate, 13: fluid chip rising and falling sealing plate, 14: jig base, 15: fluid chip inlet-side presser, 16: rising and falling guiding pin, 17: spring, 20: vacuum pump, 21: regulator, 22: filter, 23: manometer, 24: trap, 25: hand valve, 26: piping, A: fluid chip, b: fluid chip microchannel, b1: inlets, b2: outlets, c: fluid chip substrate, C1: biomolecular 1, C2: biomolecular 2, C3: biomolecular 3, C4: biomolecular 4, C5: biomolecular 5, C6: biomolecular 6, C7: biomolecular 7, C8: biomolecular 8, C9: biomolecular 9, C10: biomolecular 10, d: Pipetman or dispenser.

### BEST MODE FOR CARRYING OUT THE INVENTION

The inventors of the present invention discovered a method for ultrafast detection and measurement of biological reactions, and completed a simple and compact device system for the implementation of the method. That is, the present invention provides a method that is capable of ultrafast and precise detection of biomolecules because the effective concentration of the biomolecules immobilized on the solid-phase substrate of the micro fluid chip is high and the immobilized biomolecules maintain a high effective concentration and is prevented from flowing away even under a high flow rate. The method of the present invention comprises immobilizing a biomolecule on a substrate, placing a microchannel on the substrate, moving a fluid including a molecule with an affinity for the biomolecule at a high speed on the micro fluid chip, performing a biomolecular reaction between the biomolecule and the molecule with the affinity on the micro fluid chip, and detecting the biomolecular reaction.

The ultrafast measurement of the present invention is not possible without the high-speed moving of fluid, but in order to measure a biological reaction at an ultrafast rate using the present invention, it becomes a necessary requirement to obtain a high effective concentration by maximizing the amount of exposed area of the biomolecules immobilized on the solid-phase substrate. As a method for meeting such a requirement, it is preferable to immobilize the biomolecules so that they form a porous structure on the substrate plate. Moreover, the ultrafast measurement of the present invention may also meet two requirements: (1) a technique for strongly immobilizing proteins on the substrate so that they do not flow away even when the flow rate of the fluid is made high and (2) a technique for obtaining a large area of exposure and therefore a high effective concentration of the biomolecules immobilized on the solid-phase substrate. As an immobilization technique of proteins meeting these requirements, the electrospray deposition method is preferable.

As the method for immobilizing biomolecules so that they form a porous structure on the substrate, it is preferable to adopt the electrospray deposition method though not to be limited specifically to it. The electrospray deposition method as a method for immobilizing biomolecules is known widely in the art, and for example, the description in the PCT international publication WO 98/58745 can be referred to.

However, the technique that may be used for the immobilization of the proteins in the present invention is not to be limited to the electrospray deposition method, and pin-type spotters, inkjet, microcontact printing (Quist AP, Pavlovic E, Oscarsson S: Recent advances in microcontact printing. Annal Bioanal. Chem. 381: 591-600, 2005), immobilization method using microchannels (Cesaro-Tadic S, Demick G, Juncker D, Buurman G, Kropshofer H, Michel B, Fattinger C, Delamarche E: High-sensitivity miniaturized immunoassays for tumor necrosis factor alpha using microfluidic systems.. Lab Chip 4: 563-569, 2004) and the like may also be used.

As the immobilization substrate used in the present invention, it is preferable to use glass boards coated by indium tin oxide (ITO) or the like or various immobilization substrate plates that the surface is coated by functional groups such as aldehyde, epoxy, succinimide, maleimide, thiol, amino, carboxyl, or the like. However, the immobilization substrate is not limited to the above, and slide glass or plastic substrate plates coated by nitrocellulose, hydrophobic plastic substrate plates such as polystyrene, or substrate plates coated by hydrophilic gel may also be used for the purpose of the present invention.

Moreover, the present invention provides a micro fluid chip device system that controls the flow speed on the micro fluid chip in a fast and precise way and detects ultrafast reactions with high sensitivity. The device system of the present invention comprises a micro fluid chip including a substrate plate with immobilized biomolecules and a microchannel, an aspiration or pressurization circuit including an instrument for moving the fluid at a high speed, and a jig for connecting the above mentioned instrument for moving the fluid at a high speed and the above mentioned micro fluid chip.

Using the present invention, it was made possible to reduce the reaction time of biomolecules which used to require several minutes to several hours to only an instant to several tens of seconds and to measure and analyze biomolecular reactions in an ultrafast way. Furthermore, in the present description, "to measure biomolecular reactions at ultra high-speed" means measuring biomolecular reactions in a short amount of time of 0.01 to 60 seconds, preferably 0.1 to 20 seconds, and more preferably 0.1 to 10 seconds.

Moreover, the flow rate for moving the fluids used in the present invention is a high flow rate of 15 mm/sec to 1500 mm/sec, preferably 100 mm/sec to 1500 mm/sec, but is not limited to this range. The flow rate used varies depending on the structure of and materials used for the microchannels and the type of biomolecular reaction. Furthermore, in the present description, "moving fluids at a high speed" means moving at a flow rate mentioned above, that is a flow rate of 15 mm/sec to 1500 mm/sec, and preferably 100 mm/sec to 1500 mm/sec.

As the micro fluid chip that can be used for the purpose of implementing the present invention, it is possible to use the micro chip of JP 2002-243734 A mentioned earlier or modifications of the said micro chip according to the sample to be tested or laboratory conditions. However, the micro fluid chip used in the present invention should not be understood to be limited necessarily to the micro chip described in JP 2002-243734 A, and it is possible to use other micro chips within the scope of the technical idea of the present invention. Moreover, the term "micro fluid chip" in the present specification has the meaning commonly understood in this technical field, and concretely, it means a base plate having a size in the order of several millimeters to several centimeters squared whereon holes for inserting materials to be tested and microscopic flow channels with the width of several microns to several hundreds of microns are formed.

By using the micro fluid chip to perform biological reactions in microscopic flow channels, the efficiency of the reactions is improved, and it becomes possible to detect reactions precisely with high sensitivity in a short amount of time. The inventors of the present invention examined keenly methods of using a micro fluid chip to detect reactions precisely in an ultrafast manner, that is in a short amount of time of an instant to several tens of seconds, and completed the present invention.

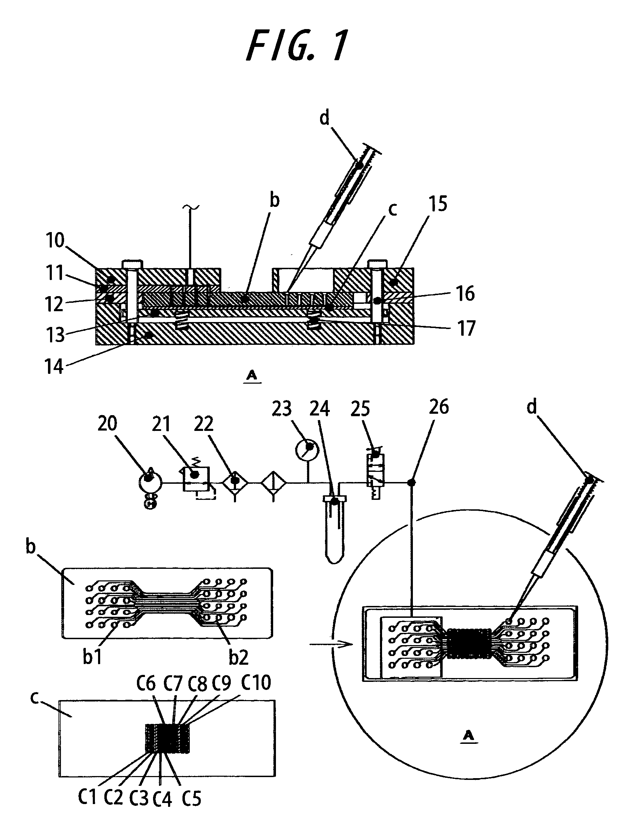

An overall view of the device system used in the method of the present invention is shown in Fig.1. The system of the present invention comprises a substrate plate with immobilized biomolecules (fluid chip substrate c in Fig. 1), microchannels (fluid chip microchannel b in Fig.l), an aspiration or pressurization circuit including an instrument for channeling the fluid at a high speed (aspiration circuit 21-25 including a vacuum pump 20 in Fig. 1), and jigs 10-17 for connecting the above mentioned instrument for flowing the fluid at a high speed and the above mentioned microchannel. An ultrafast analysis system comprising a 16-channel micro fluid chip whereon 10 types of biomolecules from biomolecule 1 to biomolecule 10 are immobilized is shown in Fig.1, but the descriptions including the number of biomolecules, the number of channels, and the pump system do not limit the scope of the present invention in any way.

The micro fluid chip is set on fluid chip jig A, and the micro fluid chip and the aspiration circuit 21-25 including the vacuum pump 20 are connected (Fig. 1). Here, the fluid chip jig A comprises a fluid chip outlet presser 10 for connecting the aspiration circuit and jigs, a fluid chip sealing plate 11 for connecting the fluid chip and the aspiration circuit, a fluid chip guiding plate 12 for aligning the fluid chip outlets, a rising and falling sealing plate 13 for setting and connecting the fluid chip, a jig base 14 for holding each jig, a fluid chip inlet-side presser 15 for holding down the fluid chip inlet, a rising and falling guiding pin 16 for guiding the fluid chip rising and falling sealing plate 13, and a spring 17 for fixing and connecting the fluid chip. However, components of jig A are not limited to the above, and may be changed as necessary.

In the aspiration circuit between the vacuum pump 20 and the micro fluid chip, a hand valve 25 for switching the pressure, a trap 24 for the waste fluid, a manometer 23 for monitoring the aspiration pressure, a filter 22 for preventing contamination, and a regulator 21 for controlling the pressure are connected by piping 26. In order to accomplish a precise control of the pressure, it is preferable to place the hand valve 25 on the side of micro fluid chip outlet b2 and the regulator 21 on the side of the vacuum pump, then making the length of the piping between the hand valve 25 and the micro fluid chip outlet b2 as short as possible. Inside of the aspiration circuit is constantly depressurized by the vacuum pump 20, and by controlling the amount of depressurization to be constant using regulator 21, it is possible to switch between constant depressurization and atmospheric pressure inside the microchannel using the hand valve 25. However, the scope of the present invention is not limited to the mechanism described above.

The fluid inserted into the micro fluid chip inlet b1 flows at a constant and high flow rate under the control of aspiration pressure by the vacuum pump 20. The aspiration pressure of the vacuum pump 20 varies according to the structure, diameter, length, and materials of the micro fluid chips b (microchannel) and c (substrate). In the example of the micro fluid chip shown in Fig. 1, the aspiration pressure is preferably controlled to be in the range of -1 kPa to -100 kPa, the value of the aspiration pressure is not limited to this range.

Moreover, it is preferable to place in the piping a trap 24 for collecting the waste fluid discharged from the micro fluid chip and a filter 22 for preventing waste fluids that leak from trap 24 from entering the vacuum pump 20.

There are dimples for holding 1-20 µl of liquid in the micro fluid chip inlet b1, and reactant solutions and rinsing solutions are dropped using a Pipetman or dispenser d, but the method for feeding liquids into the micro fluid chip and the shape of the inlet are not limited to this method.

As biomolecules to be immobilized for measurement and analysis using the micro fluid chip, there are biopolymers such as antigens, antibodies, various enzymes, receptors, ligands, various proteins, DNAs, RNAs, glucides (sugars), lipids and the like, but they are not limited to these. The biomolecular reaction detected by the present invention refers to reactions between a molecule and a molecule with biological affinity for the molecule. As concrete examples of such biomolecular reactions, reaction between an antigen and an antibody, reaction between avidin and biotin, reaction between a receptor and a ligand reacting to the receptor, reaction between an enzyme and a substrate of the enzyme, reaction between DNA and protein or DNA, reaction between RNA and protein or RNA and the like can be given. In the method of the present invention, by immobilizing one of such biomolecules with biological affinity for each other on the substrate plate and moving a fluid containing the other biomolecule at a high speed, it is possible to perform biomolecular reactions on the micro fluid chip.

After immobilizing biomolecules, it is preferable also in the present invention to perform a blocking reaction using solution of protein such as skim milk or bovine serum albumin in order to prevent test samples and the like to be flowed in later from nonspecifically adsorbing on the flow paths or substrate.

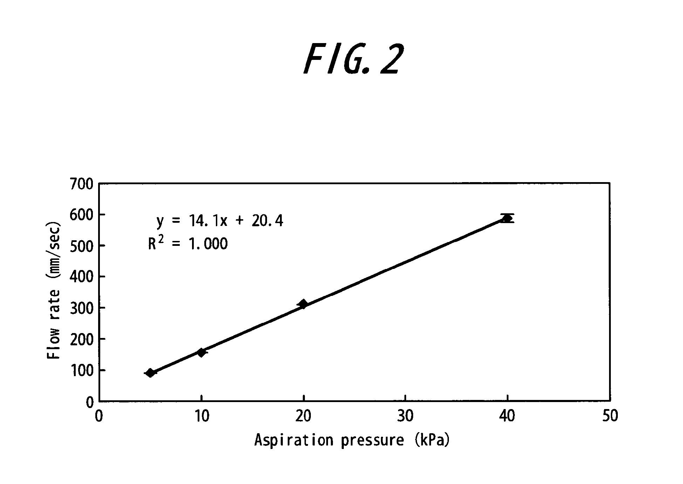

In the channels of the micro fluid chip whereon biomolecules are immobilized, a reactant solution of antigens, antibodies, ligands, substrates and the like or rinsing solution is moved at a high flow rate using a pump for pressurization or aspiration. Here, as a means of moving the fluid at a high speed, an aspiration circuit including a vacuum pump for aspiration can be used. The flow rate of the fluid can be controlled by the aspiration pressure, and the aspiration pressure used depends on the structure, bore diameter, length, and material of the microchannels, as well as the type of biomolecular reaction. As an example, flow rate control using the micro fluid chip shown in Fig. 1 is shown in Fig. 2. Furthermore, the means for moving the fluid that can be used in the present invention is not limited specifically, and other means may be adopted as necessary. As such means are given means using pumps such as aspiration pumps or pressurization pumps, means that give vibration, or means that give centrifugal force.

The detection of biomolecular reactions is done directly or through reaction of one or multiple steps as the example of detection by means of an enzyme-labeled antibody in various methods such as light absorption method, fluorescence method, emission method, fluorescence polarization method, time-resolved fluorescence method, electrochemical method and the like. In this way, a reaction of one or multiple steps, then the rinsing of the reaction solution is performed in order continuously under flow rate control, and the reaction is detected. The flow rate for each reaction is controlled by the pump, but the flow rates vary according to the type of reaction. Also, as for the reaction solution rinsing, rinsing can be performed instantly under a high flow rate, and the amount of rinsing solution and its flow rate vary according to the type of reaction.

### EXAMPLES

The present invention is described in further detail using the following embodiments and figures, but these descriptions do not limit the scope of the present invention in any way.

### (Example 1)

On a slide glass substrate plate coated with ITO was placed a glass mask 0.1 mm thick with linear openings 1 mm wide and 9.6 mm long, and 100 ng of goat-derived immunoglobulin G (IgG) (Sigma-Aldrich Corp.) was sprayed on using an electrospray deposition device. After placing microchannels produced using polydimethylsiloxane (PDMS) on this substrate plate, it was set in the aspiration pump system shown in Fig. 1.

After instant rinsing by flowing in 10 µl of 2% ECL Advance Blocking Agent-PBS solution (General Electric Company, blocking solution) per channel at 10 kPa, 10 µl of peroxidase-labeled anti-goat antibody (Jackson Immuno Research Laboratories, Inc.) diluted to 800 µg/ml with blocking solution was channeled instantly under a high flow rate control of 15.6 to 586 mm/sec, the antigen-antibody reaction was performed, and the reaction was stopped by instantly rinsing the channels with 20 µl of blocking solution. The above was done in 3 channels for each condition.

After removing the microchannels, dropping ECL Advance Western Blotting Detection Kit (Amersham Biosciences K. K.) on the substrate plate, and placing a cover slip on top, the substrate plate was filmed for 1 minute using cooled CCD camera (Bitran Corporation) in a dark place. The emission intensity of each spot in the filmed image was measured using Array-Pro Analyzer (Nippon Roper Co., Ltd., Japan).

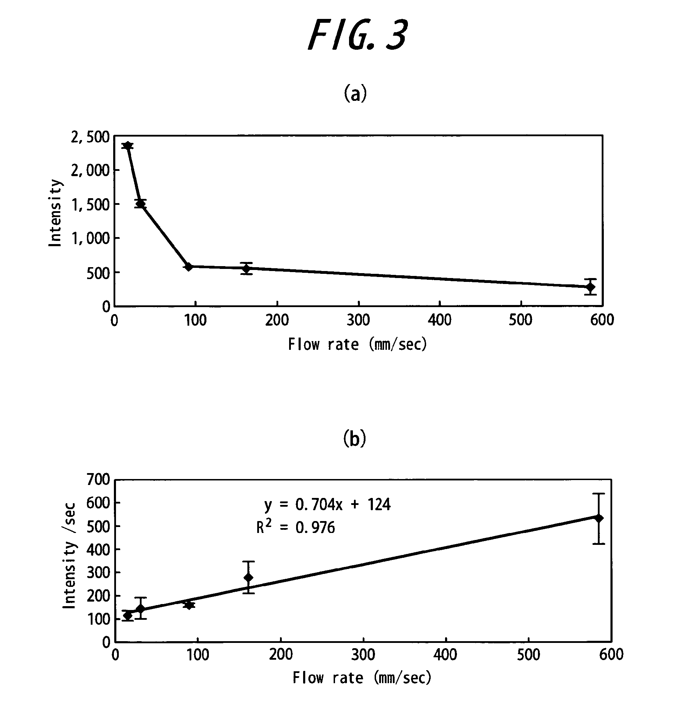

The average binding rate (emission intensity) and the binding amount per unit time was plotted for each flow rate. The standard error is shown in bars (Fig. 3). Here, Fig. 3a shows the relationship between the flow rate and the binding rate, and Fig. 3b shows the relationship between the flow rate and binding amount per unit time. As the flow rate of the antibody solution was increased, the binding rate of anti-IgG antibody to IgG decreased (Fig. 3a), but the binding amount of anti-IgG antibody per unit time increased linearly with flow rate (Fig. 3b).

### (Example 2)

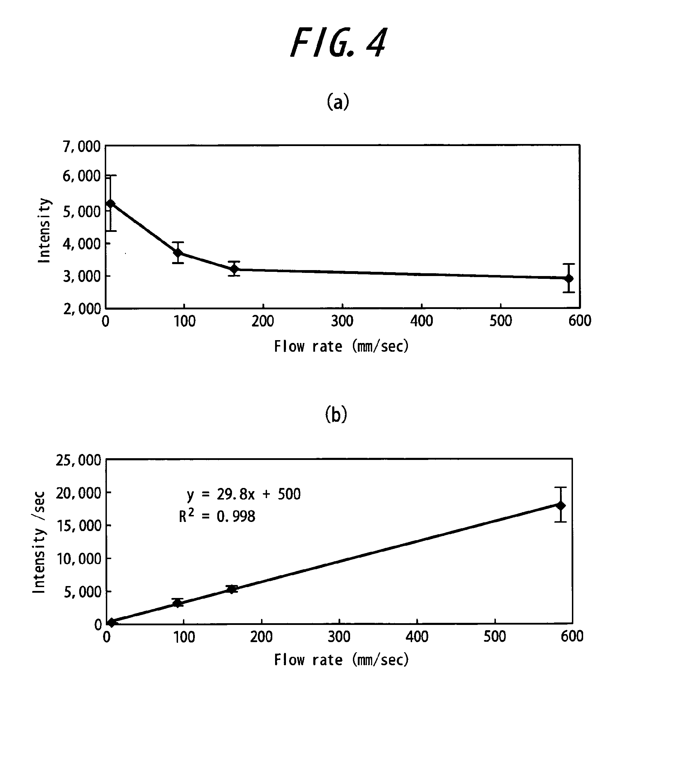

Similarly to example 1, after 100 ng of avidin (Wako Pure Chemical Industries Ltd., Japan) was sprayed onto the ITO substrate plate, rinsing was done using blocking solution, 3 µl of 5 µg/ml biotin-labeled peroxidase (Jackson Immuno Research Laboratories, Inc.) was flowed at flow rates of 5 to 586 mm/sec, and the binding reaction was performed. The reaction was stopped by rinsing the channels with 20 µl of blocking solution. The above was done in 3 channels for each condition, and the binding rate (emission intensity) and the binding amount per unit time were plotted for each flow rate (Fig. 4). Here, Fig. 4a shows the relationship between the flow rate and the binding rate, and Fig. 4b shows the relationship between the flow rate and the binding amount per unit time. In the binding reaction of biotin and avidin, as the flow rate was increased, the binding rate of biotin to avidin decreased (Fig. 4a), but the binding amount per unit time increased linearly with flow rate (Fig. 4b).

### (Example 3)

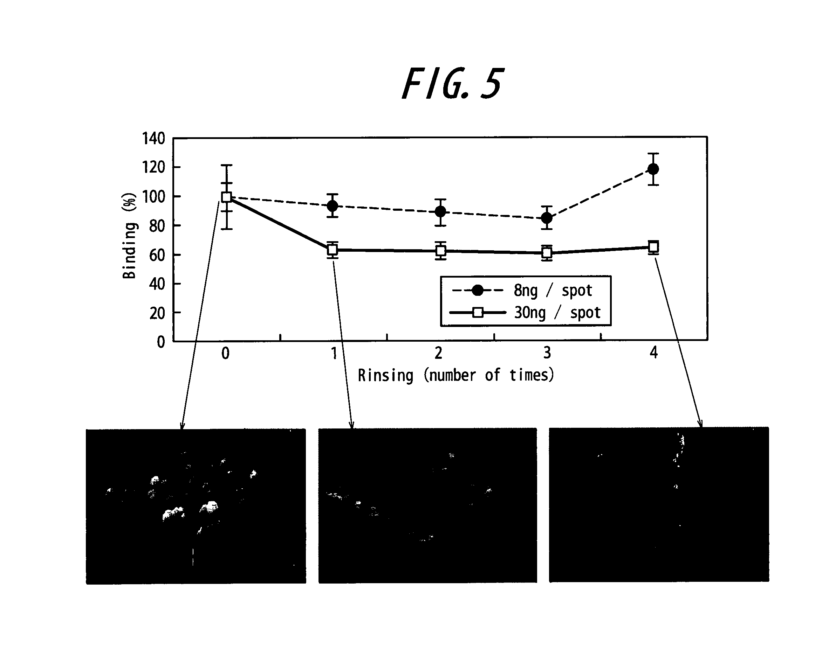

Similarly to example 2, 250 or 1000 ng (8 or 30 ng per spot) of Alexa Fluor 568 fluorochrome-labeled anti-goat antibody (Molecular Probes, Inc.) was sprayed onto the ITO-coated substrate plate using an electrospray deposition device. After setting the microchannels on this, rinsing was done 0-4 times using blocking solution. Rinsing was done in 3 channels for each condition by aspirating 20 µl of blocking solution at 10 kPa using an aspiration pump system. After rinsing, the microchannels were removed and their fluorescence was observed in a dark place using BX51WI fluorescence microscope (Olympus Corporation, Japan) and cooled CCD (Hamamatsu Photonics K.K., Japan). The amount of fluorescence of the spots was measured using Array-Pro Analyzer. For each number of times rinsing was done, the amount of fluorescence, the relative binding rate with 100% representing the value when rinsing was done zero times, and the standard error was measured and calculated (Fig. 5 upper column). Also, the surface structures of 30 ng per spot of sprayed-on fluorescence-labeled proteins after zeroth, first, and fourth rinsing were observed using NanoScope IIIa scanning probe microscope (Digital Instruments, Inc.) (Fig. 5 lower column).

As a result, as shown in Fig. 5 upper column, in the case where the electrosprayed amount is 8 ng per spot (black circle), there was no decrease in fluorescence even after four times of rinsing under a high flow rate, and no outflow of fluorescence-labeled antibody proteins was observed. In the case where 30 ng were sprayed on per spot (white square), there was a decrease in fluorescence after the first rinsing and outflow of a part of the deposited fluorescence-labeled antibodies was observed, but no outflow was detected after second and later rinsing. On the other hand, the proteins right after they are electrosprayed show a porous structure comprising particles having diameters of 10-odd nanometers to 100 and several tens of nanometers, and this structure was maintained even after rinsing (Fig. 5 lower column).

### (Example 4)

Similarly to example 2, 100 ng of the antigen of Clostridium piliforme which is a pathogen that affects mice (Missouri University Research Animal Diagnostic Laboratory, U.S.A.) was electrosprayed onto the ITO-coated substrate plate, and the microchannels were placed on top of the substrate plate and set in the aspiration pump system. After rinsing with blocking solution, 10 µl of mouse-derived Prezyme "Seiken" TZ-positive control (most positive) containing anti-C. piliforme antibody (Denka Seiken Co., Ltd., Japan) were flowed into two channels at 10kPa. Here, the flow rate was approximately 160 mm/sec, and the reaction time was 2 seconds. Blocking solution was channeled as control. After rinsing with blocking solution, 10 µl of 1 µg/ml peroxidase-labeled ProteinA (Merk-Calbiochem Ltd.) was aspirated at 10 kPa in a similar way and was made to bind with the anti-C. piliforme antibody that was immobilized onto the substrate plate. Then, similarly to example 2, the reaction was detected using enzyme chemiluminescence.

As a result, while the background average luminescence was 32 for the blocking solution, it was 204 for the positive control. In this ultrafast antigen-antibody reaction of C. piliforme antigen and the antibody reacting to the antigen, a significant signal of more than six times that of the background was obtained.

### (Example 5)

Similarly to example 2, DNA containing the DRE recognition sequence of aryl hydrocarbon receptor (Ahr) was electrosprayed and immobilized on the substrate plate. After setting the microchannels and rinsing the channels with blocking solution, a sample containing dioxin and a solution of Ahr and Ah receptor nuclear transporter protein (Arnt) was channeled in the channels. Then a complex of the DRE sequence, Ahr, Arnt, and dioxin was formed on the substrate plate. After the formation of the complex, rabbit-derived anti-Arnt antibody and peroxidase-labeled anti-rabbit antibody were reacted in order, and finally, ECL Advance was used to detect the reaction through enzyme chemiluminescence.

### INDUSTRIAL APPLICABILITY

According to the present invention, a method for ultrafast and precise measurement and analysis of biomolecules using a small sample was established, and moreover, a compact and simple micro fluid device for implementing the method was provided. Using the method and the micro fluid device of the present invention, the ultrafast, instant, and precise analysis ofbiomolecules is possible, and there is a great deal of potential for application in the research field, manufacturing field or medical field, environmental monitoring and the like.

## Claims

1. A method for measuring a biomolecular reaction at ultra-high speed, comprising the steps of: immobilizing a biomolecule on a substrate; placing a micro fluid chip on the substrate; moving a fluid containing a molecule with an affinity for the biomolecule at a high speed on the micro fluid chip; performing a biomolecular reaction between the biomolecule and the molecule with the affinity on the micro fluid chip; and detecting the biomolecular reaction.

2. A method according to claim 1, wherein the biomolecule is immobilized on the substrate such that a porous structure is formed.

3. A method according to claim 1 or 2, wherein the biomolecule is immobilized on the substrate using an electrospray deposition method.

4. A method according to any one of claims 1 to 3, wherein the substrate for immobilizing the biomolecule is a glass board coated by indium tin oxide.

5. A method according to any one of claims 1 to 4, wherein the biomolecule is a substance chosen from a group consisting of antigen, antibody, various enzymes, receptor, ligand, various proteins, DNA, RNA, glucide, and lipid.

6. A method according to claim 5, wherein the biomolecule is an antigen or an antibody.

7. A method according to any one of claims 1 to 6, wherein an instrument for moving the fluid at the high speed is an aspiration pump or pressurization pump.

8. A device system for use in the method according to any one of claims 1 to 7, **characterized in that** the fluid is moved at a high speed in the micro fluid chip for measuring a biomolecular reaction.

9. A device system according to claim 8, wherein the micro fluid chip comprises a microchannel, the device system comprises a substrate with an immobilized biomolecule, an aspiration or pressurization circuit including an instrument for moving the fluid at a high speed and a jig for connecting the instrument for moving the fluid at a high speed and the microchannel.
